Europäisches Patentamt

European Patent Office

Office européen des brevets

(11) Veröffentlichungsnummer: **0 218 544**
**A1**

(12) **EUROPÄISCHE PATENTANMELDUNG**

(21) Anmeldenummer: 86810332.6

(22) Anmeldetag: 24.07.86

(51) Int. Cl.⁴: **A 61 B 17/00**

(30) Priorität: 18.09.85 CH 4045/85

(43) Veröffentlichungstag der Anmeldung:
15.04.87 Patentblatt 87/16

(84) Benannte Vertragsstaaten:
**AT BE DE FR GB IT NL SE**

(71) Anmelder: **FRITZ GEGAUF AG**
**BERNINA-NAEHMASCHINENFABRIK**
**Seestrasse**
**CH-8266 Steckborn (CH)**

(72) Erfinder: **Dreier, Ernst**
**Mühlhofstrasse 27**
**CH-8266 Steckborn (CH)**

**Lahodny, Johann**
**Conradstrasse 36**
**A-3950 Gmünd (AT)**

(74) Vertreter: **Steiner, Martin et al**
**c/o AMMANN PATENTANWAELTE AG BERN**
**Schwarztorstrasse 31**
**CH-3001 Bern (CH)**

(54) **Chirurgisches Instrument.**

(57) Im Bereiche der Verbindungsstelle zwischen den Branchen (1, 2) und deren Ringösen (6, 7) sind Fortsätze (8, 9) mit Rastverzahnungen (11) zur Verriegelung des Instrumentes in geschlossenem, geklemmtem Zustand vorgesehen. Im Bereiche des einen Fortsatzes (9) ist ein Schieber (12) mit Keilfläche (20) vorgesehen, der durch Druck gegen eine am Schieber ausgebildete Taste (14) entgegen Federwirkung aus einer Ruhestellung zwischen die Fortsätze (8, 9) eingeschoben werden kann, um diese Fortsätze zu spreizen und die Rastverzahnungen auszurücken. In dieser Weise kann ohne erheblichen Aufwand oder Behinderung der Bedienung und in sehr wirksamer Weise ein Entriegeln des geschlossenen Instrumentes bewirkt und sein Oeffnen erleichtert werden.

FIG.1

EP 0 218 544 A1

**Beschreibung**

Chirurgisches Instrument

Die vorliegende Erfindung betrifft ein chirurgisches Instrument mit schwenkbar verbundenen Branchen, die mittels eines Rastgesperres in einer Schliesslage verriegelbar sind, und mit Hilfsmitteln zum Ausrücken des Rastgesperres.

Bekannte Instrumente dieser Art, wie Zangen, Abklemmscheren und dergleichen, erweisen sich als zu aufwendig, zu sperrig, umständlich in der Bedienung und beim Arbeiten mit dem Instrument hinderlich.

In einem Falle sind an Stelle der üblichen Ringösen zum Erfassen und Betätigen des Instrumentes plattenartige, gegenüber der Bewegungsebene der Branchen geneigte Betätigungsgriffe vorhanden, und durch verschiedenartigen Druck mit dem Daumen auf den einen dieser Griffe kann die starr mit den Branchen verbundene Rastverzahnungen in Eingriff gebracht oder entriegelt werden. Abgesehen davon, dass dieses Instrument nicht annähernd in der üblichen praktischen Weise gehandhabt und bedient werden kann, erscheint es fraglich, ob mit den vorgeschlagenen Mitteln der angegebene Zweck erreicht werde (US-PS 2 393 680).

Es ist auch bekannt, den einen mit einer Rastverzahnung versehenen Teil des Rastgesperres schwenkbar an der einen Branche anzubringen und durch Federkraft normalerweise in Eingriffsstellung zu halten bzw. zu bringen. Durch Fingerdruck kann jedoch dieser Teil des Rastgesperres entgegen der Federwirkung ausgerückt werden (US-PS 3 038 467). Diese Anordnung ist sperrig und behindert das Arbeiten. Ein weiterer Nachteil besteht darin, dass ein durch die vollen Rastkräfte belasteter Teil beweglich mit einer Branche verbunden sein muss.

Bei einem weiteren bekannten Instrument ist ein Betätigungsgriff bzw. eine Ringöse beweglich mit der ihr zugeordneten Branche verbunden, und dieser Griff oder diese Ringöse ist mit einem Teil des Rastgesperres derart gekuppelt, dass dieser Teil beim Schliessen des Instrumentes in Eingriff gebracht oder gehalten wird und beim Oeffnen des Instrumentes ausgerastet wird (DE-PS 20 25 868). Diese Ausführung ist sehr aufwendig, und sie hat den Nachteil, dass beim Klemmvorgang die volle auf den Griff oder die Ringöse wirkende Betätigungskraft auf den klinkenartig ausgebildeteten ausrückbaren Teil des Rastgesperres wirkt, womit hoher Widerstand und hoher Verschleiss entsteht.

Wie erwähnt, hat sich infolge der genannten Nachteile keine der bekannten Losungen in der Praxis durchgesetzt, und es wird daher allgemein mit Instrumenten gearbeitet, welche keinerlei Hilfsmittel aufweisen, was jeweils beim Oeffnen des Instrumentes bzw. dem Lösen des Rastgesperres Kraft und Geschick erfordert und ein feinfühliges Arbeiten erschwert.

Ziel vorliegender Erfindung ist es, eine einfache, zuverlässige Hilfsvorrichtung zum Ausrücken des Rastgesperres vorzusehen. Die Lösung ist im kennzeichnenden Teil des Anspruchs 1 umschrieben. In diesem Falle kann das Rastgesperre an und für sich in üblicher Weise ausgeführt sein und arbeiten, und es ist lediglich ein einfaches zusätzliches Organ zum Ausrücken des Rastgesperres vorzusehen. Der Keil kann an einem gegen Federkraft verschiebbaren Stössel gebildet sein. Dieser Stössel kann vorzugsweise eine Drucktaste aufweisen, die sich im Bereiche des Verbindungsteils zwischen einer der Branchen und deren Ringöse befindet. Diese Drucktaste, welche das einzige, etwas vorstehende und zugängliche Element der Hilfsmittel darstellt, ist damit an geschützter Stelle zwischen der einen Branche und deren Ringöse angebracht. Die Taste ist damit einerseits gegen ungewollte Betätigung gut geschützt und andererseits befindet sie sich an einer Stelle, wo sie die herkömmliche Bedienung des Instrumentes in keiner Weise behindert. Zudem ist sie leicht zu betätigen.

Eine besonders einfache Ausführung ist dann möglich, wenn das Rastgesperre starr mit den Branchen verbundene, mit Rastverzahnungen versehene Fortsätze aufweist, die durch Eigenelastizität der Branchen verriegelbar bzw. verriegelt sind. Es kann damit die übliche bewährte Ausführung des Instrumentes beibehalten werden und es ist lediglich ein sehr einfacher zusätzlicher Mechanismus erforderlich, um die Branchen entgegen ihrer Eigenelastizität aus dem verriegelten Zustand zu spreizen.

Die Erfindung wird nun anhand eines in der Zeichnung dargestellten Ausführungsbeispiels näher erläutert.

Figur 1 zeigt eine perspektivische Ansicht des Instrumentes,

Figuren 2 und 3 zeigen Einzelheiten des Instrumentes im grösseren Massstab, und

Figur 4 zeigt eine Teil-Ansicht von unten.

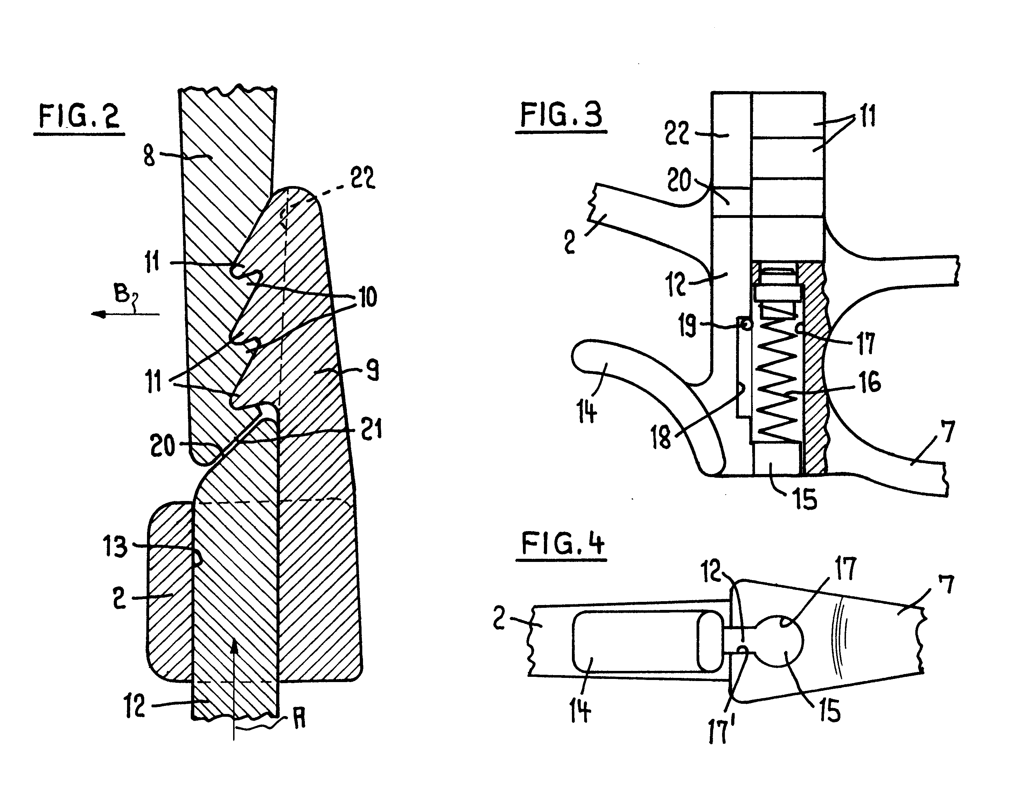

Das dargestellte Instrument weist Branchen 1 und 2 auf, die mittels eines Stiftes 3 schwenkbar gekuppelt sind. Die vorderen Enden sind als Klemmbacken 4 und 5 einer Klemme oder Zange ausgebildet. An den hinteren Enden der Branchen 1 und 2 sind Ringösen oder Griffösen 6 bzw. 7 geformt. An der Verbindungsstelle der Branchen 1 und 2 mit ihren Ringösen 6 bzw. 7 sind nach innen ragende Fortsätze 8 bzw. 9 mit je einer Rastverzahnung 10 (Figur 2) bzw. 11 vorgesehen. Soweit entspricht das Instrument der heute allgemein üblichen Ausführung. Beim Schliessen der Zange oder Klemme greifen die Rastverzahnungen 10 und 11 infolge der Eigenelastizität der Branchen 1 und 2 seitlich ineinander und verriegeln das Instrument in geschlossenem Zustand, d.h., wenn die Klemmbakken 4 und 5 ein nicht dargestelltes Gewebe zwischen sich genügend klemmen. Je nach Dicke und Beschaffenheit des Gewebes und dem ausgeübten Druck, kann die Schliess-Stellung etwas verschieden sein, was infolge der dreistufigen Rastverzahnung möglich ist. Wie erwähnt, ist es etwas mühsam, die einmal eingerasteten Verzahnungen 10 und 11 wieder zu lösen. Hiezu muss nämlich nicht nur der Druck erhöht werden, sondern die Branchen 1 und 2 müssen auch aus ihrer gemeinsamen Ebene verschoben werden, d.h., es ist eine bestimmte Verwindung der Zange erforderlich, und diese Verwindung muss aufrecht erhalten werden, bis das Instrument soweit geöffnet ist, dass die Rastverzahnungen 10 und 11 nicht mehr zum Eingriff gelangen können.

Diesem Nachteil soll die vorliegende Erfindung abhelfen. Zu diesem Zweck ist an der Verbindungsstelle zwischen der Branche 2 und ihrer Ringöse 7 eine Hilfsvorrichtung zum Ausrücken der Rastverzahnung angeordnet. Diese Vorrichtung weist einen Schieber 12 auf, dessen prismatischer Schaft in einer entsprechenden Führung 13 des Branchenkörpers längsverschiebbar geführt ist. Am äusseren Ende ist der Schieber mit einer Drucktaste 14 versehen, die relativ gut geschützt in der Einbuchtung zwischen der Branche 2 und der Ringöse 7 liegt. Dieser Taste 14 gegenüber weist der Schieber 12 einen Fortsatz auf, gegen welchen eine Druckfeder 16 abgestützt ist, die in einer Bohrung 17 der Verbindungsstelle zwischen Branche 2 und Ringöse 7 vorgesehen ist. Der Schieber 12 weist an der Innenseite im Bereiche der Feder eine Ausfräsung 18 auf, in welche ein Sicherungsstift 19 eingreift, der die äussere, in den Figuren dargestellte, unwirksame Ruhelage des Schiebers 12 bestimmt, wenn derselbe nur unter der Wirkung der Druckfeder 16 steht. Wie die Figur 4 zeigt, greift der Schieber 12 in einen Schlitz 17', welcher mit der Bohrung 17 eine hinterschnittene Nut bildet. Der Fortsatz 15 des Schiebers 12, gegen welchen die Feder 16 wirkt, ist als runde Scheibe ausgeführt, die mit wenig Spiel in die Bohrung 17 greift und damit eine zusätzlich wirksame Führung für den Schieber 12 darstellt. Am inneren Ende des Schiebers 12 ist eine Keilfläche 20 gebildet. Sie liegt bei der dargestellten, unwirksamen Ruhelage des Schiebers 12 in unmittelbarer Nähe der Branche 2 und behindert somit das volle Einrasten des Rastgesperres in die in Figur 2 dargestellte Lage nicht. Der Keilfläche 20 des Schiebers 12 liegt eine Keilfläche 21 des Fortsatzes 8 gegenüber. Seitlich der Rastverzahnung 11 des Fortsatzes 9 ist am gleichen Fortsatz eine Gleit- und Stützfläche 22 für den Schieber 12 vorgesehen. Der Schieber 12 kann also aus der dargestellten Ruhelage in Richtung des Pfeils A in Figur 2 nach innen verschoben werden, wobei seine Keilfläche 20 auf die Keilfläche 21 des Fortsatzes 8 auftrifft. Der als Keil wirkende Schieber 12 drängt hierbei die beiden Fortsätze 8 und 9 auseinander (Pfeil B) und rückt die Sperrverzahnungen 10 und 11 aus. Der Schieber oder Keil 12 ist dabei nicht irgendwie auf Biegung beansprucht, weil er stets einseitig auf der Fläche 22 und anderseitig am Fortsatz 8 abgestützt ist. Der Fortsatz 8 könnte dabei im Bereiche des Schiebers oder Keils 12 seitlich der Rastverzahnung 10 ebenfalls eine Gleit- und Stützfläche für den Schieber oder Keil 12 aufweisen.

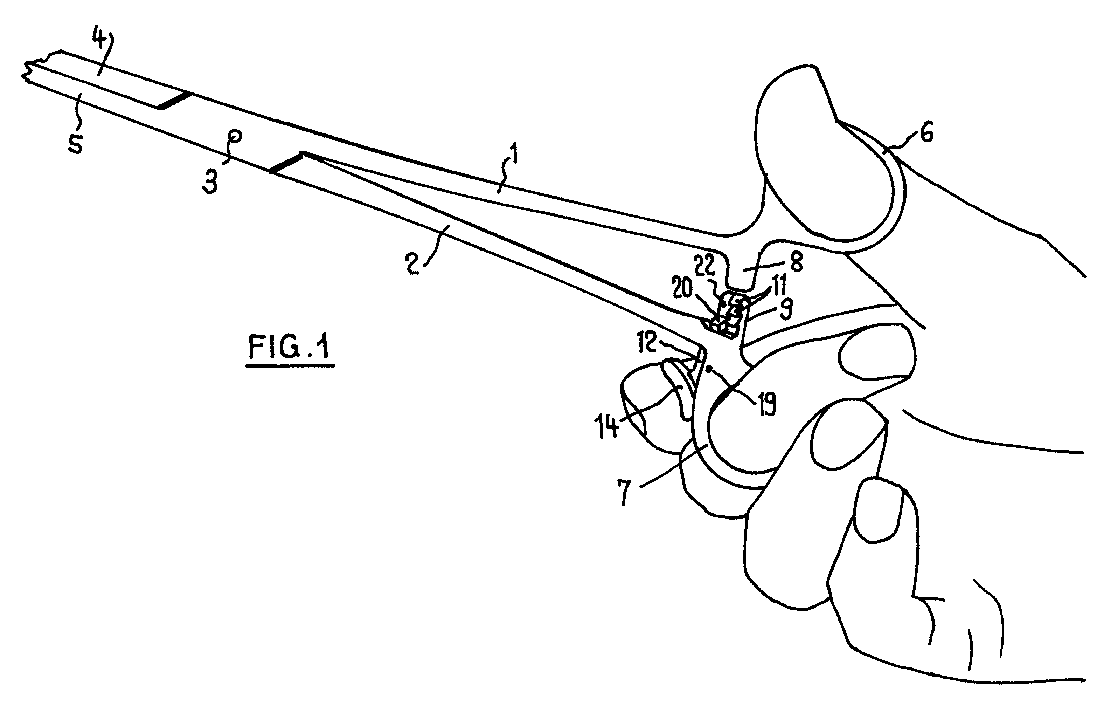

Die Bedienungsweise des Instrumentes ergibt sich bereits weitgehend aus dem Vorstehenden. In Figur 1 ist angedeutet, wie das Instrument mit dem Daumen und Mittelfinger einer Hand erfasst wird, um die Zange oder Klemme zu betätigen. Zum Ausrük-

ken des Rastgesperres bei geschlossener Zange oder Klemme, kann man bequem mit dem Zeigefinger gegen die Taste 14 drücken und damit den Schieber oder Keil 12 zwischen die Fortsätze 8 und 9 verkeilen und damit die Rastverzahnungen entgegen der Eigenelastizität der Branchen 1 und 2 ausrücken. Dann kann die Zange oder Klemme bequem mit Daumen und Mittelfinger geöffnet werden. Wird sodann die Taste 14 freigegeben, geht der Keil oder Schieber 12 unter der Wirkung der Feder 16 in seine unwirksame Ruhelage zurück, und das Instrument ist bereit für eine weitere Betätigung unter gegenseitiger Verriegelung der Branchen mittels des wirksamen Rastgesperres.

## Patentansprüche

1. Chirurgisches Instrument mit schwenkbar verbundenen Branchen (1, 2), die mittels eines Rastgesperres (8 bis 11) in einer Schliesslage verriegelbar sind, und mit Hilfsmitteln (12) zum Ausrücken des Rastgesperres, dadurch gekennzeichnet, dass als Hilfsmittel ein beweglicher Keil (20) vorgesehen ist, der zum Spreizen des Rastgesperres (8 - 11) zwischen Spreizflächen (21, 22) einführbar ist.

2. Instrument nach Anspruch 1, dadurch gekennzeichnet, dass der Keil (20) an einem gegen Federkraft (16) verschiebbaren Stössel (12) gebildet ist.

3. Instrument nach Anspruch 1 oder 2, dadurch gekennzeichnet, dass der Stössel (12) eine Drucktaste (14) aufweist, die sich im Bereich des Verbindungsteils zwischen einer der Branchen (2) und deren Ringöse (7) befindet.

4. Instrument nach einem der Ansprüche 1 - 3, dadurch gekennzeichnet, dass das Rastgesperre (8 - 11) starr mit den Branchen (1, 2) verbundene, mit Rastverzahnungen (10, 11) versehene Fortsätze (8, 9) aufweist, die durch Eigenelastizität der Branchen (1, 2) verriegelbar bzw. verriegelt sind.

5. Instrument nach einem der Ansprüche 1 - 4, wobei die Spreizflächen (21, 22) je neben einer Rastverzahnung (10, 11) an Verriegelungsorganen, z.B. Fortsätzen (8, 9) der Branchen (1, 2) vorgesehen sind.

FIG.1

FIG.2

FIG.3

FIG.4

0218544

**Europäisches Patentamt**

# EUROPÄISCHER RECHERCHENBERICHT

Nummer der Anmeldung

EP 86 81 0332

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | DE-C- 273 689 (SCHAERER AG) <br> * Figuren 1,2; Patentanspruch 1 * | 1,2 | A 61 B 17/28 |
| A,D | US-A-3 038 467 (E.J. SOVATKIN) <br> * Figuren 1-3; Patentanspruch 1 * | 1 | |
| A,D | DE-A-2 025 868 (W. PETERS) <br> * Figuren 1-5; Patentanspruch 1 * | 1 | |
| A,D | US-A-3 393 680 (P. DOMINGO) <br> * Figuren 1-4; Patentanspruch 1; Spalte 3, Zeilen 48-65 * | 1 | |

-----

RECHERCHIERTE SACHGEBIETE (Int. Cl.4)

A 61 B
B 25 B
B 26 B

Der vorliegende Recherchenbericht wurde für alle Patentansprüche erstellt.

| Recherchenort | Abschlußdatum der Recherche | Prüfer |
|---|---|---|
| DEN HAAG | 06-01-1987 | NEILL M.C. |